# EUROPÄISCHE PATENTANMELDUNG

(11) **EP 1 443 613 A1**
(43) Veröffentlichungstag der Anmeldung: **04.08.2004**
(21) Anmeldenummer: 04002378.0
(22) Anmeldetag: 03.02.2004
(51) Int. Cl.: H01R 13/72, H02G 11/02, G01R 1/04

(54) **Messvorrichtung, insbesondere für medizinische oder physiologische Messgrössen**

(30) Priorität: 03.02.2003 DE 10304340
(71) Anmelder: Soehnle-Waagen GmbH & Co. KG, 71540 Murrhardt (DE)
(72) Erfinder: Nahm, Werner, Dr., 74426 Bühlerzell (DE)

(57) **Zusammenfassung**

Eine Meßvorrichtung, insbesondere für eine oder mehrere medizinische oder physiologische Meßgrößen, besteht aus einem Bedienteil (8), das ein Gehäuse (1) umfaßt und mindestens einem Sensorgehäuse (2), das mit dem Bedienteil (8) durch ein flexibles Kabel (3) verbunden ist. Um eine derartige Meßvorrichtung zu verbessern ist in dem Gehäuse (1) eine Nut (4) zur Aufnahme des flexiblen Kabels (3) vorgesehen (Fig. 1).

## Beschreibung

Die Erfindung betrifft eine Meßvorrichtung, insbesondere für eine oder mehrere medizinische oder physiologische Meßgrößen, mit einem ein Gehäuse umfassenden Bedienteil und mindestens einem Sensorgehäuse, das mit dem Bedienteil durch ein flexibles Kabel verbunden ist.

Bei der Meßvorrichtung handelt es sich insbesondere um eine Vorrichtung zur Messung und/oder Überwachung einer oder mehrerer Meßgrößen oder Parameter, insbesondere medizinischer und/oder physiologischer Meßgrößen bzw. Parameter. Der in dem Sensorgehäuse vorgesehene Sensor dient zum Erfassen einer oder mehrerer Meßgrößen, insbesondere physikalischer, chemischer und/oder biologischer Meßgrößen, insbesondere am Körper, an der Körperoberfläche und/oder in unmittelbarer Nähe des Körpers eines Lebewesens, insbesondere eines Menschen. Das Bedienteil umfaßt ein Gehäuse. Bedienteil bzw. Gehäuse sind insbesondere als Handgerät ausgestaltet, insbesondere derart, daß das Bedienteil bzw. das Gehäuse von einer Hand gehalten werden kann. Besonders vorteilhaft ist es, wenn das Bedienteil bzw. das Gehäuse derart ausgestaltet ist, daß es in einer Hand gehalten und gleichzeitig mit einem oder mehreren Fingern oder dem Daumen dieser Hand bedient werden kann. Das Bedienteil kann während der Messung vom Benutzer in der Hand gehalten werden. Es kann allerdings auch auf eine Ablage, beispielsweise einen Tisch, in der Nähe des Benutzers abgelegt werden.

Wenn die Meßvorrichtung nicht gebraucht wird, besteht die Gefahr, daß das Kabel unbeabsichtigt überdehnt oder abgerissen wird oder daß der Sensor beschädigt wird.

Aufgabe der Erfindung ist es, eine Gefahr der Beschädigung des flexiblen Kabels und/oder des Sensors zu vermeiden.

Erfindungsgemäß wird diese Aufgabe dadurch gelöst, daß in dem Gehäuse eine Nut zur Aufnahme des flexiblen Kabels vorgesehen ist. Wenn das Gerät nicht benötigt wird, kann das flexible Kabel in die Nut eingebracht werden, insbesondere in der Nut aufgewickelt oder aufgerollt werden. Hierdurch ist das Kabel aufgeräumt und geschützt, so daß es nicht unbeabsichtigt überdehnt oder abgerissen werden kann.

Vorteilhafte Weiterbildungen sind in den Unteransprüchen beschrieben.

Vorteilhaft ist es, wenn die Nut um den gesamten Außenumfang des Gehäuses umläuft. Auf diese Weise kann das Kabel besonders einfach in die Nut eingebracht werden bzw. in der Nut aufgewickelt werden. Vorteilhaft ist es, wenn die Nut um den größten Außenumfang des Gehäuses umläuft. In diesem Fall wird die Größe des Gehäuses bestmöglich ausgenutzt.

Vorzugsweise ist in dem Gehäuse eine Aufnahme für das Sensorgehäuse vorgesehen. Hierdurch wird es ermöglicht, daß das Sensorgehäuse nach dem Aufwickeln des Kabels auf einfache Weise arretiert werden kann. Dadurch ist der Sensor geschützt, und die Gefahr einer Beschädigung des Sensors wird vermieden.

Eine weitere vorteilhafte Weiterbildung ist dadurch gekennzeichnet, daß in dem Gehäuse eine von der Nut zur Aufnahme führende weitere Nut vorgesehen ist. Alternativ kann die Aufnahme für das Sensorgehäuse unmittelbar an die Nut angrenzen.

Die Meßvorrichtung kann mehrere Sensorgehäuse und mehrere flexible Kabel umfassen. Alle flexiblen Kabel können in einer einzigen Nut aufgewickelt werden. Es ist allerdings auch möglich, mehrere Nuten vorzusehen. Vorzugsweise ist für jedes Sensorgehäuse in dem Gehäuse eine Aufnahme vorhanden.

Ausführungsbeispiele der Erfindung werden nachstehend anhand der beigefügten Zeichnung im einzelnen erläutert. In der Zeichnung zeigt
- Fig. 1: eine Meßvorrichtung mit einem Bedienteil und einem Sensorgehäuse,
- Fig. 2: eine Abwandlung der Meßvorrichtung gemäß Fig. 1 mit abgewickeltem Sensorgehäuse und
- Fig. 3: die Meßvorrichtung gemäß Fig. 2 mit aufgewickeltem Sensorgehäuse.

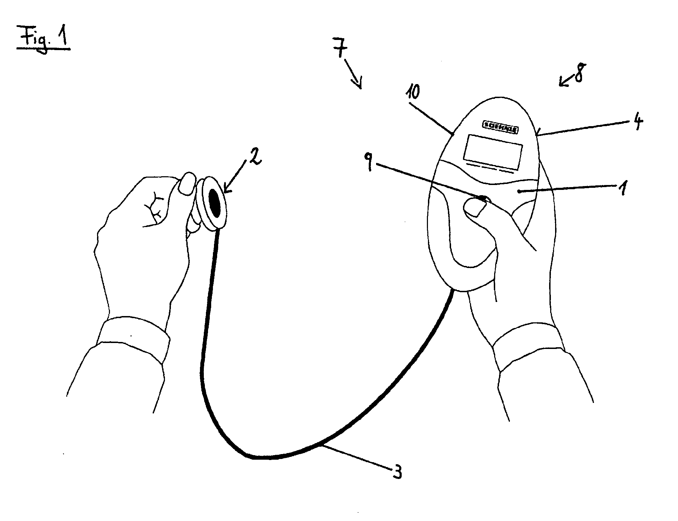

In Fig. 1 ist eine Meßvorrichtung 7 dargestellt, die aus einem Bedienteil 8, das ein Gehäuse 1 umfaßt, und einem Sensorgehäuse 2 besteht, das mit dem Bedienteil 8 durch ein flexibles Kabel 3 verbunden ist. Das Bedienteil 8 ist als Handgerät ausgestaltet. Es kann von einer Hand ergriffen werden und gleichzeitig vom Daumen dieser Hand bedient werden, indem der Daumen auf eine Taste 9 drückt. In dem Gehäuse 1 ist eine Nut 4 zur Aufnahme des flexiblen Kabels 3 vorgesehen. Die Nut 4 läuft um den gesamten Außenumfang 10 des Gehäuses 1 um, und zwar um den größten Außenumfang des abgeflachten Gehäuses 1.

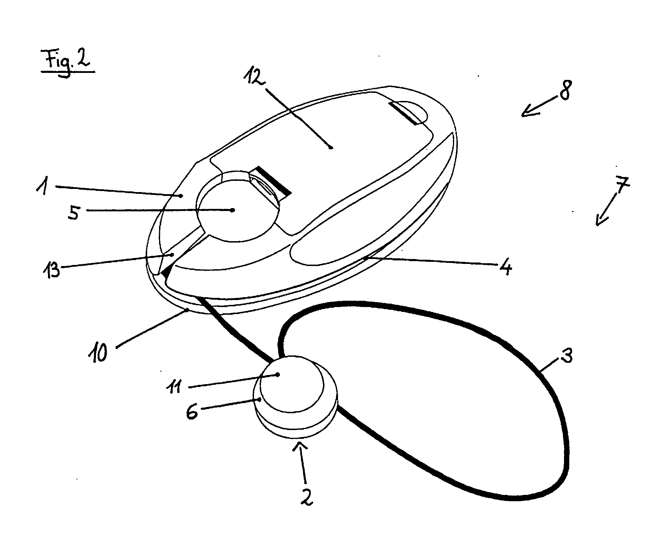

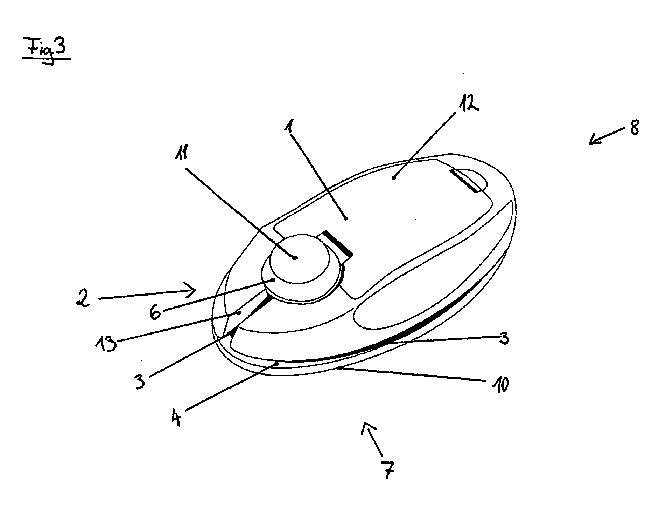

Bei der abgewandelten Ausführungsform, die in den Figuren 2 und 3 dargestellt ist, sind entsprechende Bauteile mit denselben Bezugszeichen versehen. In dem Gehäuse 1, nämlich auf dessen Oberseite, ist eine Aufnahme 5 für das Sensorgehäuse 2 vorgesehen. Die Aufnahme 5 besteht aus einer im wesentlichen zylinderförmigen Vertiefung, in welche das Unterteil 6 des Sensorgehäuses 2 in der aus Fig. 3 ersichtlichen Weise eingelegt werden kann. In dieser Stellung ist der an der Unterersichtlichen Weise eingelegt werden kann. In dieser Stellung ist der an der Unterseite des Unterteils 6 vorgesehene Sensor geschützt. Er liegt der Bodenfläche der Aufnahme 5 gegenüber und wird durch das Sensorgehäuse 2 abgedeckt. Das Oberteil 11 des Sensorgehäuses 2 ragt über die Oberfläche 12 der Oberseite des Gehäuses 1 hinaus. Das Sensorgehäuse 2 kann an dem Oberteil 11 ergriffen und aus der Aufnahme 5 herausgenommen werden.

In dem Gehäuse 1 ist ferner eine von der Nut 4 zur Aufnahme 5 führende weitere Nut 13 vorgesehen, die ebenfalls einen Teil des flexiblen Kabels 3 aufnimmt.

Die Aufnahme des Kabels 3 erfolgt durch Aufwickeln auf das Gehäuse 1. Der gesamte Außenumfang, nämlich der größte Außenumfang 10, des Gehäuses 1 wird ausgenutzt. Die Nut 4 befindet sich in diesem größten Außenumfang des Gehäuses 1. Sie ist in der Gehäuseoberfläche integriert und führt das Kabel 3 beim Aufwickeln. Das Kabel 3 verschwindet vollständig in der Nut 4. Im Gehäuse 1 des Bedienteils 8 ist eine Aufnahmevorrichtung 5 für das Sensorgehäuse 2 integriert, so daß das Sensorgehäuse 2 nach dem Aufwickeln des Kabels 3 einfach arretiert werden kann. Dadurch ist der Sensor geschützt, und gleichzeitig wird das aufgewickelte Kabel 3 arretiert.

## Patentansprüche

1. Meßvorrichtung, insbesondere für eine oder mehrere medizinische oder physiologische Meßgrößen, mit einem ein Gehäuse (1) umfassenden Bedienteil (8) und mindestens einem Sensorgehäuse (2), das mit dem Bedienteil (8) durch ein flexibles Kabel (3) verbunden ist,
**dadurch gekennzeichnet,**
**daß** in dem Gehäuse (1) eine Nut (4) zur Aufnahme des flexiblen Kabels (3) vorgesehen ist.

2. Meßvorrichtung nach Anspruch 1, **dadurch gekennzeichnet, daß** die Nut (4) um den gesamten Außenumfang (10) des Gehäuses (1) umläuft.

3. Meßvorrichtung nach Anspruch 1 oder 2, **dadurch gekennzeichnet, daß** in dem Gehäuse (1) eine Aufnahme (5) für das Sensorgehäuse (2) vorgesehen ist.

4. Meßvorrichtung nach Anspruch 3, **dadurch gekennzeichnet, daß** in dem Gehäuse (1) eine von der Nut (4) zur Aufnahme (5) führende weitere Nut (13) vorgesehen ist.

5. Meßvorrichtung nach einem der vorhergehenden Ansprüche, **dadurch gekennzeichnet, daß** die Meßvorrichtung mehrere Sensorgehäuse und mehrere flexible Kabel umfaßt.
